# EUROPEAN PATENT SPECIFICATION

(11) **EP 2 373 367 B1**
(45) Date of publication and mention of the grant of the patent: **21.10.2015**
(21) Application number: 09831036.0
(22) Date of filing: 02.12.2009
(51) Int. Cl.: A61M 11/00

(54) **SYSTEMS AND METHODS FOR DELIVERY OF A BREATHING GAS WITH FINE ICE PARTICLES**
SYSTEME UND VERFAHREN ZUR ABGABE EINES ATEMGASES MIT FEINEN EISPARTIKELN
SYSTÈMES ET PROCÉDÉS POUR LA DISTRIBUTION D'UN GAZ RESPIRATOIRE COMPRENANT DE FINES PARTICULES DE GLACE

(30) Priority: 02.12.2008 US 119305 P
(43) Date of publication of application: 12.10.2011
(73) Proprietor: Qool Therapeutics, Inc., Menlo Park, CA 94025 (US)
(72) Inventor: BELSON, Amir, Los Altos CA 94024 (US); OHLINE, Robert, M., Los Altos CA 94024 (US); TZORI, Nimrod, Los Altos CA 94024 (US)
(74) Representative: Kazi, Ilya
(86) International application number: PCT/US2009/066380
(87) International publication number: WO 2010/065616

(56) References cited:
- DE-A1-102007 019 616
- US-A1- 2002 023 640
- US-A1- 2003 131 844
- US-A1- 2007 123 813
- US-A1- 2008 015 543
- US-A1- 2008 262 377
- US-B1- 6 547 811

## Description

The present invention relates generally to system and methods for selective modification and control of a patient's body temperature. More particularly, it relates to a respiratory system and methods for raising and lowering a patient's body temperature by heat exchange with the patient's airways and lungs. The respiratory system provides rapid induction of therapeutic hypothermia by having the patient breathe a respiratory gas that carries with it a frozen mist of particles to enhance heat capacity. Known systems for using cooled gas to induce therapeutic hypothermia in a patient are disclosed in US 2003/131844 and US 2008/0262377 and for stopping nosebleeds in US 2008/0015543. The system of US 2008/0262377 includes an ice particle generator comprising a fluid source and fluid injector for introducing fine ice particles into an apparatus for inducing therapeutic hypothermia and a breathing mask for the patient to breathe through, attached to the apparatus by a hose.

In accordance with the present invention, there is provided a cold breathing gas delivery system as claimed in claim 1. The system comprises a delivery device adapted having an exit port at a distal end thereof, to deliver a cooled breathing gas mixture to a patient; wherein the delivery device (102, 302, 402, 502) is a breathing mask or an endotracheal tube sized for insertion into an airway of a patient; and an injection device positioned at or near the distal end or exit port of the delivery device. The injection device is configured to release a fluid to form a frozen mist of fine ice particles into the cooled breathing gas mixture when the breathing gas mixture is carried by the delivery device.

In a preferred embodiment, the cold breathing gas delivery system has an endotracheal tube adapted to deliver a cooled breathing gas mixture to a patient; the injection device positioned near a distal end of the endotracheal tube.

In another preferred embodiment, the cold breathing gas delivery system has a nasal cannula adapted to deliver a cooled breathing gas mixture to a patient; the at least one injection device positioned near a distal end of the nasal cannula, the at least one injection device being configured to release a fluid to form a frozen mist of fine ice particles in the cooled breathing gas mixture.

In another preferred embodiment, the cold breathing gas delivery system includes a tube sized to deliver a cooled breathing gas mixture to a throat of a patient and the injection device is positioned near a distal end of the tube.

In another preferred embodiment, a cold breathing gas delivery system includes a breathing mask adapted to deliver a cooled breathing gas mixture and the injection device is positioned near a distal end of the breathing mask.

In accordance with the present invention, there is provided a therapeutic treatment system having a delivery device adapted to deliver a cooled breathing gas mixture to a patient and an injection device positioned near a distal end of the delivery device, the injection device coupled to a source of liquid. The treatment system also includes a control system coupled to the delivery device and the injection device. Alternatively, the control system is adapted to control the injection device to release a fluid into the cooled breathing gas mixture to form a frozen mist of fine ice particles in the cooled breathing gas mixture.

In any of the embodiments described herein, the cold breathing gas delivery system can further include a control system to actuate injection of fluid from the injection device into the cold breathing gas mixture. In addition, the dispensing of fluid should be timed to the flow of the breathing gas mixture into the patient during the inhalation portion of the patient's breathing cycle. The control system can receive a sensor input that indicates when inhalation is about to occur, when it is occurring, and/or when inhalation is completing. One or more of a variety of sensors, which may comprise pressure sensors or flow sensors may be used. The control system and sensors can also record and monitor the patient's temperature using any known way of measuring a patient's temperature, such as an oral, urethral, skin, IR, or rectal probe. The control system can use the measured temperatures and pressure/flow sensors as feedback to adjust the temperature of the breathing gas mixture, the temperature of the fluid, the rate and volume of breathing gas mixture delivered to the patient, and the volume of fluid injected into the breathing gas mixture by the injection device according to the desired patient temperature.

In preferred embodiments, the apparatus provides a method for treating a patient by delivering a cooled breathing gas mixture to a target tissue within the patient and injecting a fluid into the cooled breathing gas mixture at or near the target tissue to form a frozen mist of fine ice particles in the cooled breathing gas mixture. The target tissue may be a nasal airway of the patient, a lung of the patient, a mouth and a nasal airway of the patient, a throat of the patient a trachea of the patient or any combination of the above target tissue sites.

In any of the embodiments described herein, there is also provided means for injecting a drug into or mixing with the cooled breathing gas mixture. In one aspect, the drug is anesthetic drug.

In a preferred embodiment, the cooled breathing gas mixture is delivered directly to a target tissue of the patient and is combined with a therapeutic drug to directly treat the target tissue. The anesthetic or therapeutic drug can be added to the cooled breathing gas mixture and the mixture delivered to the sinuses of a patient. The drug may be selected to treat a condition of the patient using the sinuses. The condition may be migraine headache. The condition may be to induce therapeutic hypothermia through the sinuses.

In addition, drugs can also be added to the gas mixture, either at the breathing gas source, or with the use of a separate nebulizer or the like. Drugs such as bronchodilators, local (inhaled) vasodilators or any other medications that will increase the blood flow to the lungs for better heat transfer and prevent bronchoconstriction may also be added. Additionally or alternatively, drugs that encourage perspiration, peripheral vasodilators, and drugs that reduce or eliminate shivering can be delivered with the cooled breathing gas mixture, hi addition, anesthetic drugs can be added to the breathing gas mixture or administered directly to the patient to reduce or eliminate pain in a target location that may be associated with inhaling the cooled breathing gas mixture may also be added. Anti-shivering agents and/or anti-thermoregulatory response agents may be administered to the patient to assist in achieving the desired degree of hypothermia. Alternatively or in addition, external warming, such as with a warm air blanket or electric blanket, may be applied to reduce shivering while internal hypothermia is maintained. Regional heating of selected portions of the patient's body may be used to control shivering and/or to modulate the body's thermoregulatory responses.

In preferred embodiments, the apparatus provides a method for treating a patient by forming a breathing gas mixture; cooling the breathing gas mixture; delivering the cooled breathing gas mixture to a target tissue within the patient; and injecting a fluid into the cooled breathing gas mixture at or near the target tissue to form a frozen mist of fine ice particles in the cooled breathing gas mixture. In this or any other embodiment of the invention, the system may be adapted to provide for pressurizing the breathing gas mixture and the methods adapted to include one or more steps including the use of a pressurized breathing gas mixture. The pressure of the gas mixture can also be controlled. Pressurizing the gas mixture will further improve the mass flow rate, and hence the heat transfer rate. The gas mixture should be pressurized to levels known safe to the patient (for example 1.5-2 atmospheres). Alternatively, the pressure of the gas mixture can be pulsed, i.e. vary the pressure continuously from high to low, to help mix the breathing gas and improve the heat transfer rate.

In an additional embodiment, the forming step also includes forming the breathing gas mixture from a gas mixture that includes oxygen and a gas with a high heat capacity. The breathing gas mixture may be formed from a gas mixture that includes oxygen and helium; a gas mixture that includes oxygen and carbon dioxide; a gas mixture that includes sulfur hexafluoride; or any combination of the above gas mixtures.

In another embodiment, the breathing gas mixture may be air or a special gas mixture that includes oxygen (about 20% concentration or more) and a gas with a high heat capacity (Cp) for more effective heat exchange. The mixture can be regular or purified air, or air with a higher concentration of oxygen (from 20 to 100%). An alternative gas mixture could be oxygen and helium, such as HELIOX, which is 20% oxygen and 80% helium. Since the specific heat capacity for helium is much higher than the specific heat capacity for air, such a mixture could improve the heat flow rate and enable a more effective way of lowering the patient's temperature. In another embodiment, the gas mixture can include sulfur hexafluoride SF6, which is a dense, nontoxic gas that has a much higher specific heat capacity than air. Additionally, carbon dioxide (CO2) may be added to the gas mixture to help regulate the patient's respiration rate. Nitrous oxide can also be added to the breathing gas mixture 103. The gas mixtures listed above and other combinations of biocompatible gasses that are safe for inhalation may optionally be used.

In any of the embodiments of the invention, injection into the cooled breathing gas mixture is provided with one or a combination of a fluid injector, a water injector, an air-water airbrush, a nozzle atomizer, a shaker bottle, a microfluidic device, a jet impact device, an ultrasonic droplet nozzle, a steam feeder, an ice shaver, an ultrasonic nebulizer nozzle, a swirl jet nozzle, an impaction pin nozzle, a colliding jets nozzle, a MEMS nozzle array mist generator, an electro spray nozzle, a heated capillary, an internal mixing nozzle, and an external mixing nozzle.

The novel features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings of which.
Fig. 1A illustrates a diagram of a system for delivering a frozen mist of fine ice particles to a patient.
Fig. 1B illustrates yet another embodiment of a system for delivery a frozen mist of fine ice particles to a patient.
Fig. 1C illustrates yet another embodiment of a system for delivery a frozen mist of fine ice particles to a patient.
Figs. 2A-2G illustrate various embodiments of injection devices for delivery a frozen mist of fine ice particles to a patient.
Figs. 3A-3C illustrate yet another embodiment of a system for delivery a frozen mist of fine ice particles to a patient.
Figs. 4A-4B illustrate yet another embodiment of a system for delivery a frozen mist of fine ice particles to a patient.
Figs. 5A-5B illustrate yet another embodiment of a system for delivery a frozen mist of fine ice particles to a patient.

### DETAILED DESCRIPTION OF THE INVENTION

Certain specific details are set forth in the following description and figures to provide an understanding of various embodiments of the invention. Certain well-known details, associated electronics and devices are not set forth in the following disclosure to avoid unnecessarily obscuring the various embodiments of the invention. Further, those of ordinary skill in the relevant art will understand that they can practice other embodiments of the invention without one or more of the details described below. Finally, while various processes are described with reference to steps and sequences in the following disclosure, the description is for providing a clear implementation of particular embodiments of the invention, and the steps and sequences of steps should not be taken as required to practice this invention.

The system described herein is used to create a fine mist of frozen ice particles in a cooled breathing gas mixture and deliver the cooled breathing gas mixture to a target tissue within a patient. The term "ice" as used herein should be understood to include any substance that has undergone a phase change from a vapor or liquid state to a solid state, such as by cooling. In one embodiment, the cooled breathing gas mixture and fine mist of frozen ice particles are delivered to the airways of a patient and ultimately the lungs to induce therapeutic hypothermia in the patient. The temperature of the blood in the lungs will decrease when exposed to the cold breathing gas mixture, which lowers the temperature of the heart tissue. The chilled blood can continue to flow to the coronary arteries where it will continue to lower the temperature of the tissue. In the case of myocardial infarction, the effect of this chilled blood flowing directly into the coronaries is especially beneficial. The blood can also flow from the left heart to the entire body to change the body temperature as desired. In the case of a stroke, a portion of the cooled blood will flow to the brain, cooling the tissue and reducing the metabolism and the oxygen consumption therein to reduce ischemic damage to the brain. Once therapeutic hypothermia has been achieved, the rate of heat transfer can be modulated by adjusting the quantity of ice particles delivered and the temperature of the breathing mixture can be adjusted to achieve and maintain the desired body temperature.

In another embodiment, the cooled breathing gas mixture is delivered directly to a target tissue of the patient, and can be combined with a therapeutic drug to directly treat the target tissue. For example, an anesthetic drug can be added to the cooled breathing gas mixture and the mixture can be delivered to the sinuses of a patient to treat migraine and also induce therapeutic hypothermia through the sinuses, for example.

The system described herein is designed to be portable and compact such that it can be easily administered to a patient by a variety of medical personnel, such as paramedics or EMT's in an ambulance, a medical team outside a hospital, an emergency room medical team or at any other location where this treatment is necessary. Advantages of the system include ease of operation and the ability to operate with minimal training. Thus treatment of the patient can begin much sooner after a heart attack, stroke or other event compared to other more invasive methods that can only be performed in an emergency room or in a cath lab. Rapid treatment for these conditions has been shown to improve patient outcomes by reducing ischemic damage and necrosis in the affected tissue.

The system described herein will generally include a delivery device and an injection device. The delivery device is sized for insertion into an airway of a patient, and can be an endotracheal tube, oropharyngeal airway (OPA), laryngeal mask airway (LMA), nasal cannula or nasopharyngeal airway (NPA), breathing mask, or other related medical devices. Although a typical breathing mask can be used, a slightly modified version that doesn't allow for a cold breathing gas mixture to contact the cheeks, lips, teeth, etc, of the patient is more desirable. For example, a modified breathing mask could include a short tube, such as 1-2" in length, that extends into the mouth and is held in place with a gentle biting action or natural closing tension of the jaw or lips. The type of delivery device used may depend on the personal intended to use the system. For example, only highly trained medical personnel, such as medical doctors and physician assistants, may be qualified to intubate a patient with an endotracheal tube. So a system utilizing an endotracheal tube type delivery device may be limited to a hospital setting. However, a system designed for use by an EMT or ambulance paramedic may use a breathing mask or nasal cannula as the delivery device. In general, many types of delivery devices may be used with the system to provide therapeutic hypothermia to a patient and/or treat a target tissue of the patient depending on the qualifications and location of those administering treatment.

The system can utilize various methods to create a frozen mist of fine ice particles in the patient. This can include several types of fluid injectors, ice scrapers, pressure nozzles, and the like to disperse a fine mist of fluid into a cold breathing gas mixture carried by the delivery device. When the fine mist of fluid comes into contact with the cold breathing gas mixture, a fine mist of frozen ice particles are formed and carried into the patient.

The system can additionally include a fluid source, a breathing gas mixture source, a control system, temperature and pressure sensors, pumps, and a mechanical respirator/ventilator for use with the delivery device and injection device. These system components can work together to control delivery of the cold breathing gas mixture and fine mist of frozen ice particles to the patient.

The amount of ice particles added to the breathing gas mixture is preferably in the range of 0 to 5 liters per hour (measured as the volume of fluid injected to produce the frozen mist.) A flow rate of ice particles in the range of 0.25 to 1 liters per hour is currently thought to be sufficient for rapidly achieving hypothermia in an adult human patient. Due to the latent heat of fusion (the heat required to effect a phase change from liquid water to ice), the incoming breathing gas may need to be cooled to a temperature significantly below the freezing point to achieve effective freezing of the fluid droplets. Optionally, the fluid injection can be timed with the pulsatile flow of breathing gas.

The frozen mist can carried into the patient's lungs by the breathing gas. The ice particles can melt within the patient's lungs providing a high rate of heat transfer for cooling the lungs and the blood that flows through them. Because of the high heat transfer rate provided by the melting of ice particles, an extremely low temperature will not be needed for effective cooling of the patient, thereby mitigating the risk of freezing damage to the patient's lungs. After the need for protective hypothermia has passed, the system may be used for rewarming the patient to normothermia. For example, the system may be used to inject water into the patient at a temperature higher than the body temperature but below the threshold of damage or discomfort to the patient, preferably at 37-52°C.

The amount of fluid that forms in the lungs from the melting of the ice particles can be easily tolerated by the patient. An adult human with good lung function can readily clear 1 liter per hour of fluid from the lungs through normal processes. Thus, a flow rate of ice particles in the range of 0.25 to 1 liters per hour will be readily tolerated for an extended period of several hours. Higher flow rate of ice particles, up to 5 liters per hour, can be tolerated for shorter periods. If desired, positive pressure ventilation may be used to help drive the fluid from the lung passages into the surrounding tissue and from there into the bloodstream. In addition, diuretics or other medications to treat pulmonary edema may be administered to the patient to help eliminate excess water if needed.

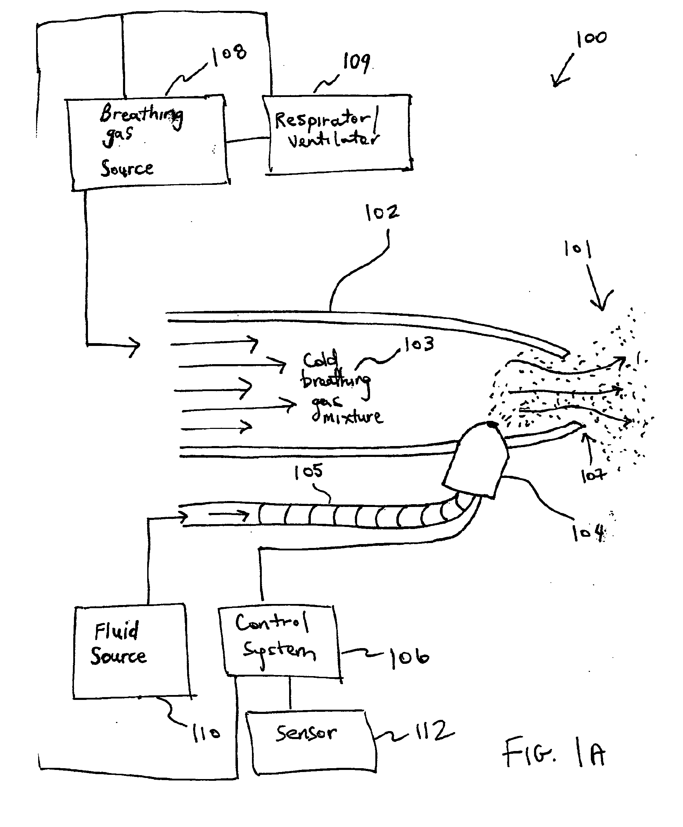

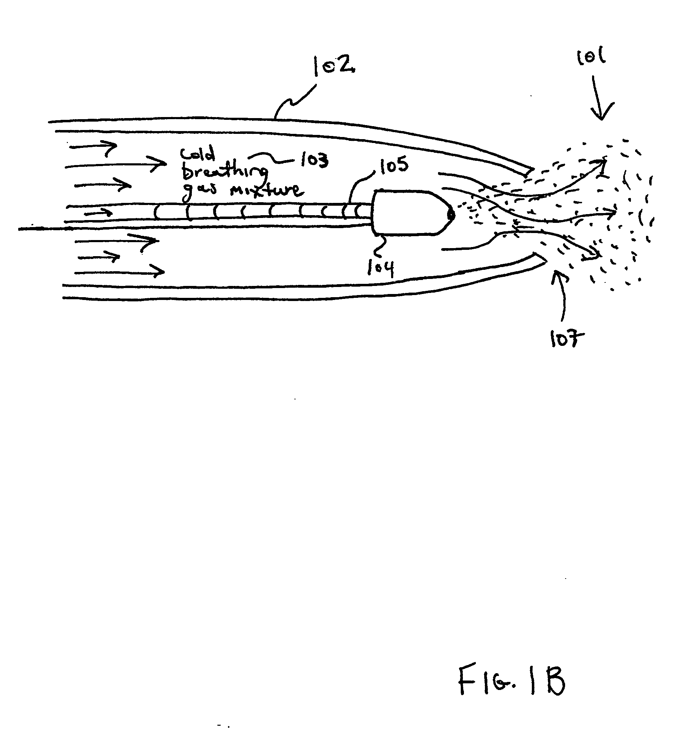

The invention will now be described with respect to the drawings. Fig. 1A illustrates a cold gas delivery system 100 comprising delivery device 102, injection device 104, control system 106, breathing gas source 108, and fluid source 110. System 100 can be adapted to be inserted into the airways of a patient, such as the mouth, nose, throat, or lungs, for treatment at a target tissue within the patient, and can be utilized to induce therapeutic hypothermia for treating a variety of conditions, including acute myocardial infarction, migraine, and emergent stroke.

Fig. 1A includes an illustration of a distal region of delivery device 102, which is adapted to deliver a breathing gas mixture 103 to a target tissue within a patient, specifically an airway of the patient such as a lung, a nasal airway, the sinuses, the throat/trachea, or the mouth. Delivery device 102 can be an endotracheal tube, oropharyngeal airway (OPA), laryngeal mask airway (LMA), nasal cannula or nasopharyngeal airway (NPA), or other related medical devices. Although delivery device 102 is illustrated in Fig. 1A as a tube or cannula type breathing gas delivery device, it can additionally be a breathing mask that is fitted over the mouth and/or nose. Delivery device 102 is coupled to a breathing gas source 108, which is adapted to supply and deliver the cold breathing gas mixture 103 through the delivery device and out exit port 107 to the target tissue of the patient. Optionally, the gas source may be connected to a mechanical respirator/ventilator 109, particularly for patients who are not breathing spontaneously.

The gas mixture 103 can be cooled using any known method of cooling. These cooling methods can be incorporated into the gas source 108 or can be separate system components. For example, heat exchangers, electric coolers, pressurization, refrigeration and the like can be utilized in system 100 to cool the gas. The heat exchanger can utilize a refrigeration cycle, a reversible heat pump, a thermoelectric heater/cooler, dry ice, liquid nitrogen or other cryogen, or other known heater/cooler to achieve the desired temperature. Similarly, gas source 108 can be submerged in a chilled liquid bath, such as an antifreeze/water bath or liquid nitrogen bath. The gas mixture 103 can be chilled to very cold temperatures, such as temperatures as low as -100°C, for example. Due to the low temperatures of gas mixture that can flow through delivery device 102, it may be necessary to insulate delivery device 102. This can be accomplished by any insulation methods as known in the art, such as fiberglass, foam, or by using evacuated doubled walled chambers.

The gas mixture 103 may be air or a special gas mixture that includes oxygen (about 20% concentration or more) and a gas with a high heat capacity (Cp) for more effective heat exchange. The mixture can be regular or purified air, or air with a higher concentration of oxygen (from 20 to 100%). An alternative gas mixture could be oxygen and helium, such as HELIOX, which is 20% oxygen and 80% helium. Since the specific heat capacity for helium is much higher than the specific heat capacity for air, such a mixture could improve the heat flow rate and enable a more effective way of lowering the patient's temperature. In another embodiment, the gas mixture can include sulfur hexafluoride SF₆, which is a dense, nontoxic gas that has a much higher specific heat capacity than air. Additionally, carbon dioxide (CO₂) may be added to the gas mixture to help regulate the patient's respiration rate. Nitrous oxide can also be added to the breathing gas mixture 103. The gas mixtures listed above and other combinations of biocompatible gasses that are safe for inhalation may optionally be used.

The pressure of the gas mixture can also be controlled. Pressurizing the gas mixture will further improve the mass flow rate, and hence the heat transfer rate. The gas mixture should be pressurized to levels known safe to the patient (for example 1.5-2 atmospheres). Alternatively, the pressure of the gas mixture can be pulsed, i.e. vary the pressure continuously from high to low, to help mix the breathing gas and improve the heat transfer rate.

Drugs can also be added to the gas mixture 103, either at the breathing gas source 108, or with the use of a separate nebulizer or the like. Drugs such as bronchodilators, local (inhaled) vasodilators or any other medications that will increase the blood flow to the lungs for better heat transfer and prevent bronchoconstriction due to the cold breathing mixture. Furthermore, drugs that encourage perspiration, peripheral vasodilators, and drugs that reduce or eliminate shivering can be delivered with the cooled breathing gas mixture. In addition, anesthetic drugs can be added to the breathing gas mixture or administered directly to the patient to reduce or eliminate pain in a target location that may be associated with inhaling the cooled breathing gas mixture. Anti-shivering agents and/or anti-thermoregulatory response agents may be administered to the patient to assist in achieving the desired degree of hypothermia. Alternatively or in addition, external warming, such as with a warm air blanket or electric blanket, may be applied to reduce shivering while internal hypothermia is maintained. Regional heating of selected portions of the patient's body may be used to control shivering and/or to "trick" the body's thermoregulatory responses.

System 100 further comprises injection device 104 configured to inject a fluid into the cold breathing gas mixture 103 to form a frozen mist of fine ice particles 101 in the cooled breathing gas mixture. Injection device 104 is preferably positioned at or near the distal end or exit port 107 of delivery device 102. Positioning the injection device close to the exit port of the delivery device maximizes the ability of system 100 to deliver a frozen mist of ice particles directly to the target tissue of the patient while maximizing the percentage of particles that exit the device and make contact with target tissue. In contrast, introducing the liquid to the breathing gas mixture at any point further upstream of the exit port could lead to ice particles adhering to the inner walls of the delivery device and causing system congestion or clogging, reducing the number of ice particles ultimately reaching the target tissue of the patient for therapeutic treatment or causing complete system failure. Even if the frozen mist of fine ice particles is formed as close to the target tissue of the patient as possible, there still may be some ice formation within the system. Thus, it may be necessary to clear ice from within the system. This can be accomplished by a variety of mechanical ways, including scrapers, wipers, brushes, by vibration at low frequencies, sonic frequencies, ultrasonic frequencies, or through the application of heat, either by heating the delivery device directly or periodically allowing a warm breathing gas mixture to flow into the device to melt any accumulations.
Injection device 104 can be coupled to fluid source 110 by fluid line 105. The fluid source could contain normal saline solution (0.9% NaCl) or any other desired solution, so that it will be isotonic with the patient's blood. However, in accordance with the present invention, water, e.g. distilled water, is used. Preferably NaCl is added to the breathing mixture, or may be administered to the patient orally or via another route to maintain an isotonic concentration. The fluid source may be held at a higher pressure than the breathing gas mixture to aid in mist formation.

In Fig. IA, the injector device 104 and fluid line 105 are positioned external to the delivery device until the point where the injector device is attached to the delivery device. It may be helpful to pre-cool the fluid within fluid source 110 to a temperature close to freezing before it is injected into the breathing gas mixture to aid in formation of fine ice particles when the fluid is released into the cold breathing gas mixture. An additional heat exchanger may be included for this purpose. Alternatively, injection device 104 and fluid line 105 may be positioned within the delivery device, as shown in Fig. IB, to provide for a more compact system. When the fluid line is positioned within the delivery device, it may be necessary to heat the fluid line to prevent the fluid from freezing within the fluid line, since the cold breathing gas mixture surrounding the fluid line can reach temperatures as low as -100°C. This heating can be accomplished by wrapping the fluid line with a coil of resistive wire, as shown in Figs. 1A-1B. Other heating embodiments may include heating the fluid within the fluid source 110, for example. The amount of heating should be as minimal as possible to avoid adversely heating the breathing gas mixture and inhibiting the system 100 from being able to form a fine mist of frozen ice particles in the breathing gas mixture.

Injection device 104 can comprise a variety of devices adapted inject fluid into the cooled breathing gas mixture. For example, injection device 104 can be a water injector, a spray bottle/nozzle atomizer, a shaker bottle, a microfluidic injector, a jet impact nozzle, an ultrasonic nozzle, a steam feeder, an ultrasonic nebulizer, a swirl jet nozzle, an impaction pin nozzle, a colliding jets nozzle, a MEMS nozzle array mist generator, an electro spray nozzle, a heated capillary, an internal mixing nozzle, an external mixing nozzle, or other appropriate injectors as known in the art. Instead of using an injection device, ice particles or fluid/mist can also be introduced into the gas mixture with an air-water airbrush (i.e., a venturi) or an ice shaver. Many of these embodiments of injection devices will be discussed in further detail below and in the drawings.

System 100 can further include a control system 106 to actuate injection of fluid from the injection device into the cold breathing gas mixture. Preferably, the dispensing of fluid should be timed to the flow of the breathing gas mixture into the patient during the inhalation portion of the patient's breathing cycle. To achieve this result, the control system can receive a sensor input that indicates when inhalation is about to occur, when it is occurring, and/or when inhalation is completing. This can be achieved by a variety of sensors 112, which may comprise pressure sensors or flow sensors. The control system and sensors 112 can also record and monitor the patient's temperature using any known way of measuring a patient's temperature, such as an oral, urethral, skin, IR, or rectal probe. The control system can use the measured temperatures and pressure/flow sensors as feedback to adjust the temperature of the breathing gas mixture, the temperature of the fluid, the rate and volume of breathing gas mixture delivered to the patient, and the volume of fluid injected into the breathing gas mixture by the injection device according to the desired patient temperature.

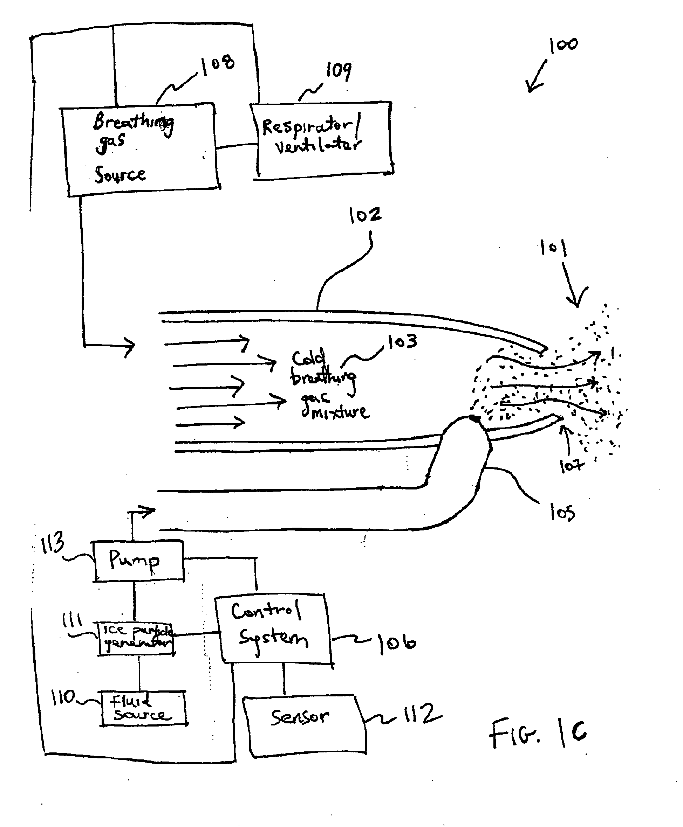

Yet another embodiment of system 100 is shown in Fig. 1C. The system can include a control system 106, breathing gas source 108, ventilator 109, fluid source 110, and sensor 112 as described above. In addition, the fluid source is connected to an ice particle generator 111 and a pump 113. The ice particle generator can utilize different methods of ice particles generation, such as mist injection into cold environment, or ice scraping, for example. As needed, ice particles can be pumped into the breathing gas mixture flow through line 105 to create a frozen mist laden flow that can be administered to the patient.

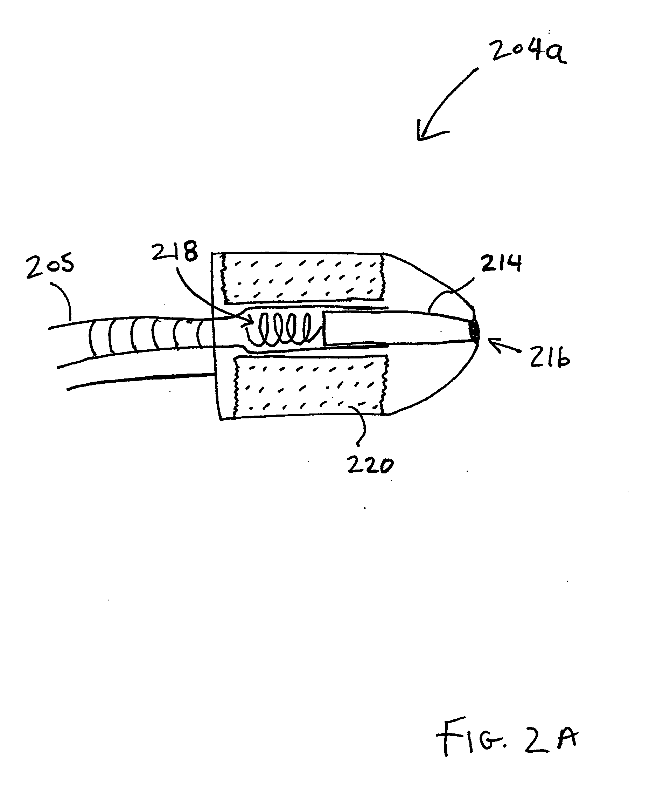

A first embodiment of an injection device suitable for use with system 100 is illustrated in Fig. 2A. Injection device 204a comprises fluid line 205, plunger 214, exit point 216, return spring 218, and solenoid coils 220. Injection device 204 can be coupled to a control system, such as with electrical leads connecting the control system to the solenoid coils. Fluid line 205 can be coupled to a fluid source, as described above. In operation, return spring 218 causes plunger 214 to apply force to and seal exit port 216 when solenoid coils 220 are not energized. However, plunger 214 is drawn proximally towards fluid line 205 when solenoid coils 220 are energized, which allows fluid to flow through the fluid injector and out through exit port 216 into the flow of the breathing gas mixture. As described above, the dispensing of fluid should be timed to the flow of the breathing gas mixture into the patient during the inhalation portion of the patient's breathing cycle. Thus, the control system can be designed to actuate the energizing of the solenoid coils to release fluid into the patient during inhalation.

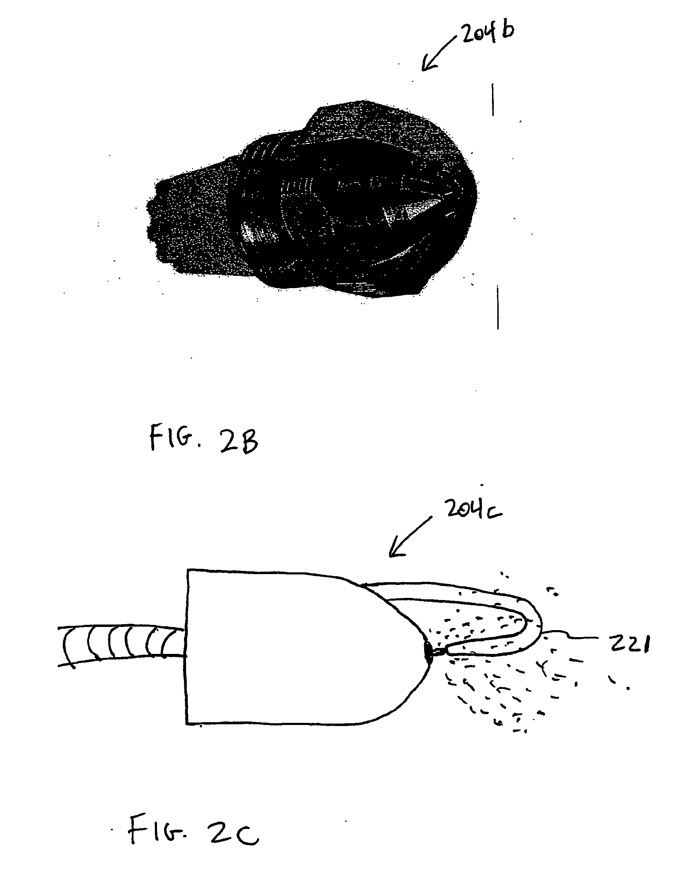

Other injection device embodiments are shown in Figs. 2B-2G. A swirl jet nozzle 204b as shown in Fig. 2B can be used with system 100 described above. Fluid can be pressurized into a cavity within the swirl jet. The internal cavity design of the swirl jet causes the fluid to accelerate and rotate. As the fluid exits the swirl jet in a spiral motion, the centrifugal forces combined with a pressure drop at the exit of the nozzle cause the fluid to break into a mist.

In Fig. 2C, an impaction pin nozzle 204c is illustrated that can be used with system 100. Fluid exits the nozzle at a high velocity and impacts the pin 221, causing the fluid to break up into a fine mist cloud. In addition to a pin, the nozzle can be designed to cause the high velocity fluid to impact a flat plate or a contoured plate to scatter the fluid and create mist.

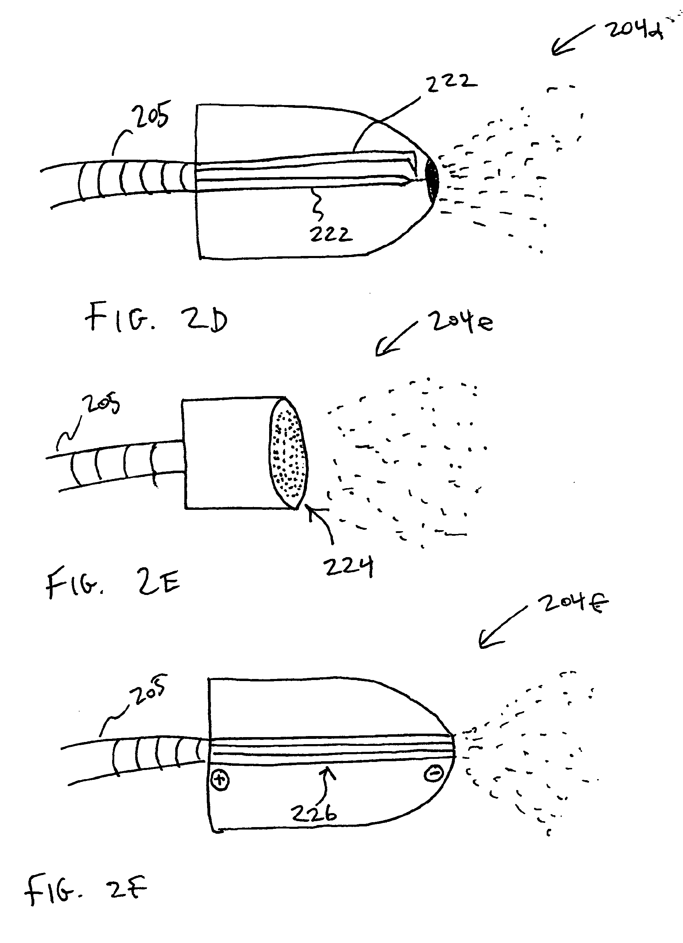

In Fig. 2D a colliding jets nozzle 204d is illustrated. Fluid received from a fluid source is divided into multiple channels 222. The channels are positioned so that fluid exiting the channels will collide, which breaks the respective fluid streams into small droplets to form a mist.

Fig. 2E illustrates a shaker bottle injector 204e comprising a perforated element 224 having very small holes therethrough. A control system, as described above, can cause the perforated element to vibrate, which allows fluid on one side of the perforated element to propagate through the small holes to form a mist. The shaker bottle injector embodiment can be manufactured in very small sizes using micro electro mechanical systems (MEMS) technology to make perforations in a membrane.

In the embodiment illustrated in Fig. 2F, a nozzle design incorporating an electro-osmotic membrane having microfluidic channels 226 can be used to create fine droplets or mist. When a positive charge is applied by a control system to a proximal end of a microfluidic channel, and a negative charge is applied to a distal end of the microfluidic channel, water flows across the electro-osmotic membrane and into the breathing gas mixture as small droplets or mist.

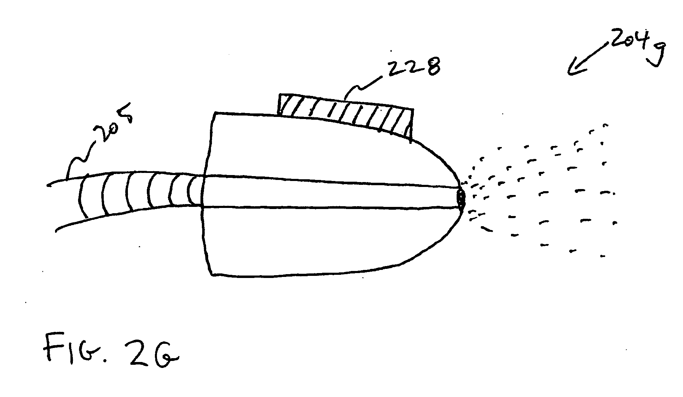

Additionally, ultrasonic nozzle 204g in Fig. 2G uses an ultrasonic transducer 228 to break up a fluid into fine mist droplets as the fluid exits the nozzle.

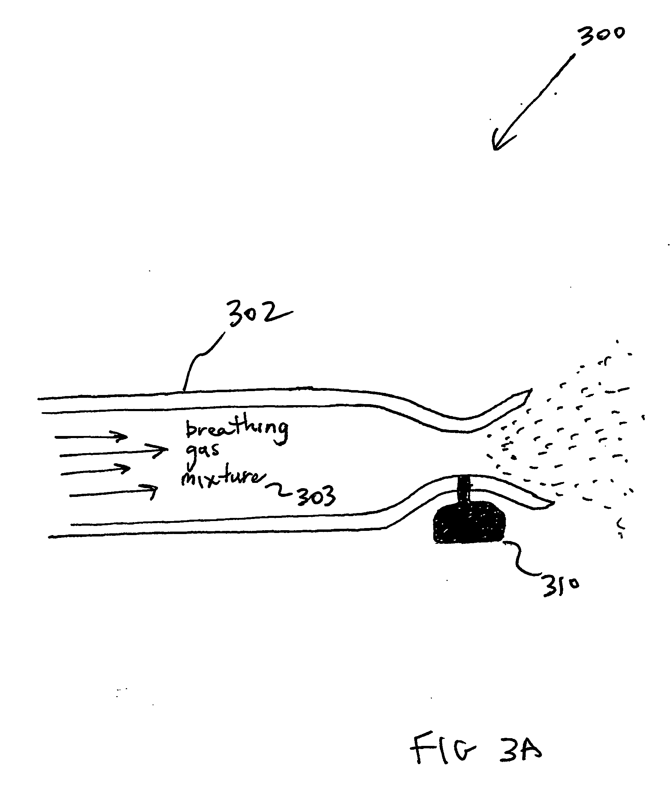

System 300 and 300' as illustrated in Figs. 3A-3B provide additional methods of forming a mist of fine ice particles in a breathing gas. System 300 and 300' can include all the system components described above, such as a fluid source, a breathing gas source, a respirator/ventilator, a control system, and sensors, but these elements have been omitted from Figs. 3A-3B for ease of illustration. In Fig. 3A, delivery device 302 is shaped as a venturi element. The venturi element has a section of reduced cross-sectional area. When the breathing gas mixture 303 flows through the venturi, its velocity increases and pressure decreases. The lower pressure draws fluid from fluid source 310 into the venturi to mix with air, which causes droplets or a fine mist to form in the gas mixture. As described above, the temperature of the gas mixture is low enough to cause formation of fine ice particles when a mist or fluid droplets are introduced to the gas mixture.

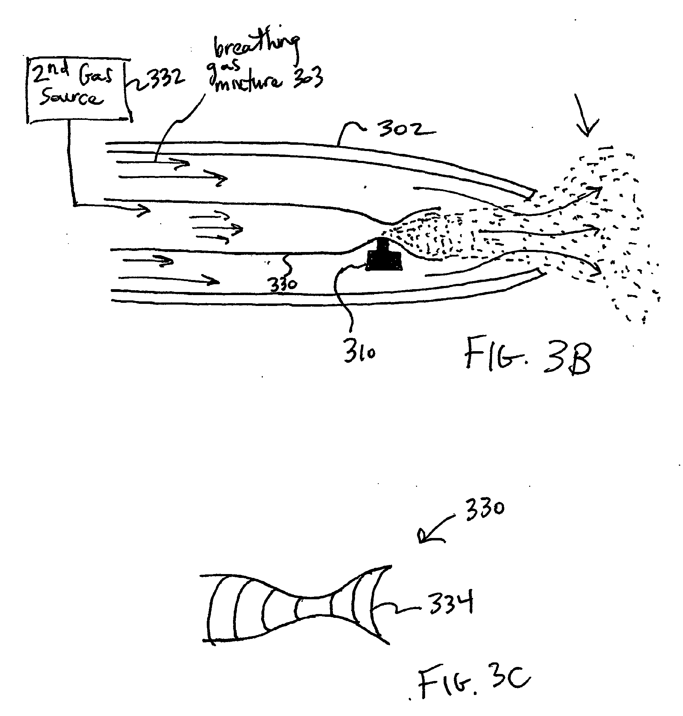

Similarly, system 300' in Fig. 3B also utilizes a venturi element to produce a fine mist of frozen ice particles in the breathing gas mixture. System 300' additionally includes a venturi element 330 positioned within the delivery device 302, and a second gas source 332 to deliver a second gas mixture through the venturi element. System 300' creates two separate air flow passageways, the first passageway carrying the bulk of breathing gas mixture 303 useful for inhalation by the patient. The second air passageway (i.e., venturi 330), is used to create the mist of water droplets to be frozen into fine ice particles. As shown in Fig. 3C, it may be advantageous to heat venturi element, such as with a coil of resistive wires 334, to prevent ice formation and system degradation within the venturi element and delivery device.

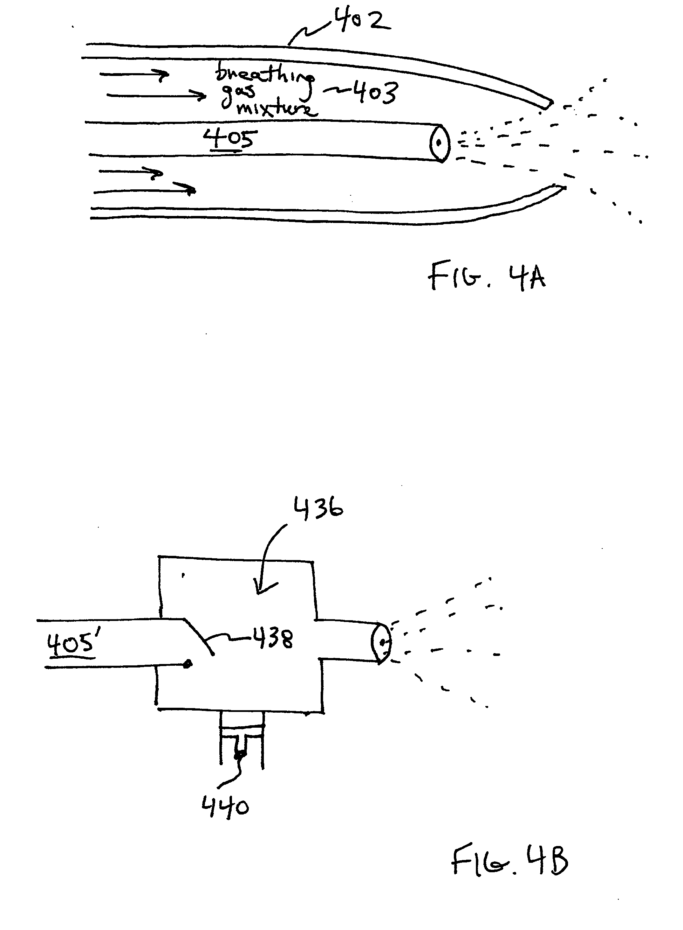

Yet another method for forming a frozen mist of fine ice particles is illustrated in Figs. 4A-4B. In Fig. 4A, fluid line 405 runs partially along the interior of delivery device 402. When the fluid within fluid line 405 is pressurized to a sufficiently high pressure, the fluid is forced through the fluid line into the breathing gas mixture and forms a mist or fine spray of fluid droplets. An embodiment of the fluid line in Fig. 4B utilizes the same concept, but additionally adds an electro-mechanically actuated piston 440, a 1-way valve 438, and a fluid reservoir 436 to control the spray. When the piston is retracted, the 1-way valve is opened and the high pressure fluid flows through and forms a mist in the surrounding breathing gas mixture. When the piston is extended, the 1-way valve is closed and no mist is formed. This embodiment can be utilized with the control system described above, for example, to time the formation of mist with inhalation by the patient.

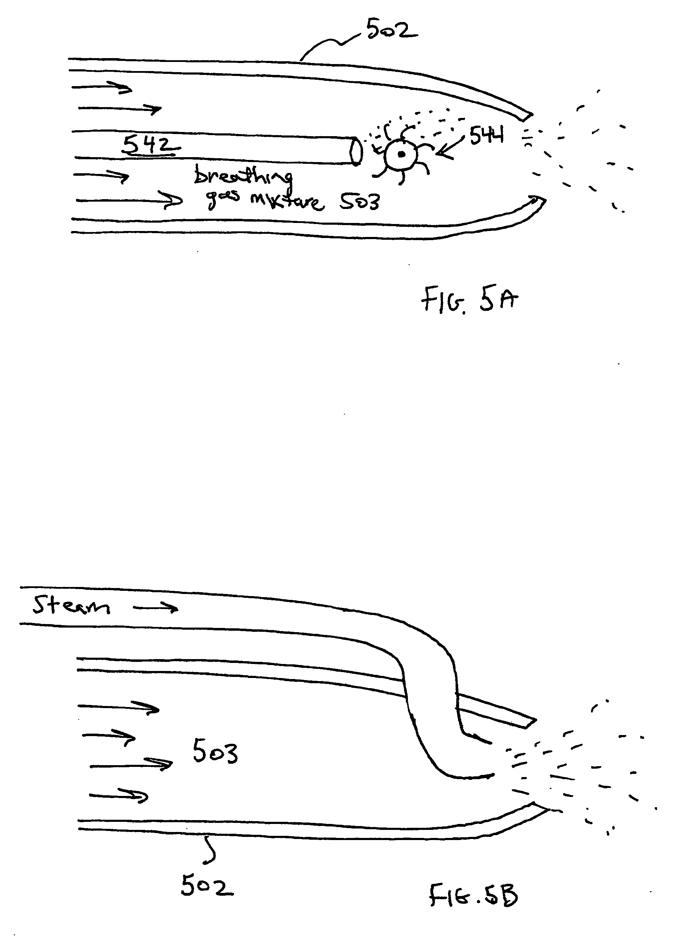

Figs. 5A-5B illustrate additional ways to form a fine mist of frozen ice particles in a breathing gas. In Fig. 5A, ice shavings, ice chunks, or small ice particles are carried by ice line 542 to ice scatterer 544, which further breaks the ice into smaller pieces or particles. The fine ice mist is then carried by the cold breathing gas mixture into the patient as described above. In an alternative embodiment (not illustrated), ice shavings can be delivered from outside the patient to the delivery device with a vibro feeder. An example of a vibro feeder is a vibrating element that "walks" materials down an assembly line in manufacturing. A compact version of this system could be adapted and configured to deliver ice particles to a patient through the delivery device, for example.

Fig. 5B uses steam to create a fine mist of frozen ice particles. Steam fed into a very cold breathing gas mixture will result in the formation of water droplets which can further be cooled to freeze into a fine mist of frozen ice particles. It may be necessary to cool the breathing gas mixture 503 to temperatures lower than that described in the above embodiments, since the steam will introduce heat into the breathing gas mixture. These ice particles can then be delivered to the patient in the cold breathing gas mixture.

As for additional details pertinent to the present invention, materials and manufacturing techniques may be employed as within the level of those with skill in the relevant art. The same may hold true with respect to method-based aspects of the invention in terms of additional acts commonly or logically employed. Also, it is contemplated that any optional feature of the inventive variations described may be set forth and claimed independently, or in combination with any one or more of the features described herein. Likewise, reference to a singular item, includes the possibility that there are plural of the same items present. More specifically, as used herein and in the appended claims, the singular forms "a," "and," "said," and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation. Unless defined otherwise herein, all technical and scientific terms used herein have the same meaning as commonly understood by one of ordinary skill in the art to which this invention belongs. The breadth of the present invention is not to be limited by the subject specification, but rather only by the plain meaning of the claim terms employed.

## Claims

1. A cold breathing gas delivery system (100), comprising;
a delivery device (102, 302, 402, 502) having an exit port (107) at a distal end thereof, adapted to deliver a cooled breathing gas mixture (103, 303, 403, 503) to a patient; and
an injection device (104, 204, 330, 544) positioned at or near the distal end or exit port (107)_of the delivery device **characterised in that** the injection device is configured to release a fluid to form a frozen mist of fine ice particles into the cooled breathing gas mixture when the breathing gas mixture is carried by the delivery device (102, 302, 402, 502) and wherein the delivery device (102, 302, 402, 502) is a breathing mask or an endotracheal tube sized for insertion into an airway of a patient.

2. The cold breathing gas delivery system (100) of claim 1 wherein the injection device comprises a fluid injector.

3. The cold breathing gas delivery system (100) of claim 1 wherein the injection device comprises an air-water airbrush.

4. The cold breathing gas delivery system (100) of claim 1 wherein the injection device comprises a nozzle atomizer.

5. The cold breathing gas delivery system (100) of claim 1 wherein the injection device comprises a shaker bottle.

6. The cold breathing gas delivery system (100) of claim 1 wherein the injection device comprises a microfluidic device.

7. The cold breathing gas delivery system (100) of claim 1 wherein the injection device comprises a jet impact device.

8. The cold breathing gas delivery system (100) of claim 1 wherein the injection device comprises an ultrasonic droplet or nebulizer nozzle.

9. The cold breathing gas delivery system (100) of claim 1 wherein the injection device comprises a steam feeder.

10. The cold breathing gas delivery system (100) of claim 1 wherein the injection device comprises an ice shaver.

11. The cold breathing gas delivery system (100) of claim 1 wherein the injection device comprises a swirl jet nozzle.

12. The cold breathing gas delivery system (100) of claim 1 wherein the injection device comprises an impaction pin nozzle.

13. The cold breathing gas delivery system (100) of claim 1 wherein the injection device comprises a colliding jets nozzle.

14. The cold breathing gas delivery system (100) of claim 1 wherein the injection device comprises a MEMS nozzle array mist generator.

## Patentansprüche

1. Abgabesystem (100) für kaltes Atemgas, wobei das System Folgendes umfasst:
eine Abgabevorrichtung (102, 302, 402, 502), die eine Austrittsöffnung (107) an einem distalen Ende davon aufweist, die dafür ausgelegt ist, ein gekühltes Atemgasgemisch (103, 303, 403, 503) an einen Patienten abzugeben; und
eine Einspritzvorrichtung (104, 204, 330, 544), die an oder nahe dem distalen Ende oder der Austrittsöffnung (107) der Abgabevorrichtung angeordnet ist, **dadurch gekennzeichnet, dass** die Einspritzvorrichtung dafür konfiguriert ist, ein Fluid freizugeben, um einen gefrorenen Nebel von feinen Eispartikeln in dem gekühlten Atemgasgemisch zu bilden, wenn das Atemgasgemisch durch die Abgabevorrichtung (102, 302, 402, 502) befördert wird,
und wobei die Abgabevorrichtung (102, 302, 402, 502) eine Atemmaske oder ein Endotrachealtubus ist, die bzw. der zum Einsetzen in einen Atemweg eines Patienten dimensioniert ist.

2. Abgabesystem (100) für kaltes Atemgas nach Anspruch 1, wobei die Einspritzvorrichtung einen Fluidinjektor umfasst.

3. Abgabesystem (100) für kaltes Atemgas nach Anspruch 1, wobei die Einspritzvorrichtung eine Luft-Wasser-Spritzpistole umfasst.

4. Abgabesystem (100) für kaltes Atemgas nach Anspruch 1, wobei die Einspritzvorrichtung einen Düsenzerstäuber umfasst.

5. Abgabesystem (100) für kaltes Atemgas nach Anspruch 1, wobei die Einspritzvorrichtung eine Schüttelflasche umfasst.

6. Abgabesystem (100) für kaltes Atemgas nach Anspruch 1, wobei die Einspritzvorrichtung eine Mikrofluidvorrichtung umfasst.

7. Abgabesystem (100) für kaltes Atemgas nach Anspruch 1, wobei die Einspritzvorrichtung eine Strahlschlagvorrichtung umfasst.

8. Abgabesystem (100) für kaltes Atemgas nach Anspruch 1, wobei die Einspritzvorrichtung eine Ultraschalltröpfchen- oder Zerstäuberdüse umfasst.

9. Abgabesystem (100) für kaltes Atemgas nach Anspruch 1, wobei die Einspritzvorrichtung eine Dampfzuführung umfasst.

10. Abgabesystem (100) für kaltes Atemgas nach Anspruch 1, wobei die Einspritzvorrichtung einen Eisrasierer umfasst.

11. Abgabesystem (100) für kaltes Atemgas nach Anspruch 1, wobei die Einspritzvorrichtung eine Verwirbelungsdüse umfasst.

12. Abgabesystem (100) für kaltes Atemgas nach Anspruch 1, wobei die Einspritzvorrichtung eine Prallstiftdüse umfasst.

13. Abgabesystem (100) für kaltes Atemgas nach Anspruch 1, wobei die Einspritzvorrichtung eine Kollisionsstrahldüse umfasst.

14. Abgabesystem (100) für kaltes Atemgas nach Anspruch 1, wobei die Einspritzvorrichtung einen MEMS- Düsenanordnungsnebelerzeuger umfasst.

## Revendications

1. Système de distribution d'un gaz respiratoire froid (100), comprenant :
un dispositif de distribution (102, 302, 402, 502) possédant un orifice de sortie (107) au niveau d'une extrémité distale de celui-ci, conçu pour distribuer un mélange de gaz respiratoire refroidi (103, 303, 403, 503) à un patient ; et
un dispositif d'injection (104, 204, 330, 544) positionné au niveau de l'extrémité distale ou de l'orifice de sortie (107), ou à proximité de ces postes, du dispositif de distribution, **caractérisé en ce que** le dispositif d'injection est configuré de façon à dégager un fluide pour former un brouillard gelé de fines particules de glace dans le mélange de gaz respiratoire refroidi lorsque le mélange de gaz respiratoire est acheminé par le dispositif de distribution (102, 302, 402, 502), et cas dans lequel le dispositif de distribution (102, 302, 402, 502) est un masque respiratoire ou une sonde d'intubation endotrachéale dimensionnée en vue d'une insertion dans une voie aérienne d'un patient.

2. Système de distribution d'un gaz respiratoire froid (100) selon la revendication 1, le dispositif d'injection comprenant un injecteur de fluide.

3. Système de distribution d'un gaz respiratoire froid (100) selon la revendication 1, le dispositif d'injection comprenant un aérographe air/eau.

4. Système de distribution d'un gaz respiratoire froid (100) selon la revendication 1, le dispositif d'injection comprenant un atomiseur à buse.

5. Système de distribution d'un gaz respiratoire froid (100) selon la revendication 1, le dispositif d'injection comprenant une bouteille à agitation.

6. Système de distribution d'un gaz respiratoire froid (100) selon la revendication 1, le dispositif d'injection comprenant un dispositif microfluidique.

7. Système de distribution d'un gaz respiratoire froid (100) selon la revendication 1, le dispositif d'injection comprenant un dispositif d'impact par jets.

8. Système de distribution d'un gaz respiratoire froid (100) selon la revendication 1, le dispositif d'injection comprenant une buse de nébulisation ou à gouttelettes ultrasonique.

9. Système de distribution d'un gaz respiratoire froid (100) selon la revendication 1, le dispositif d'injection comprenant un dispositif d'alimentation de vapeur.

10. Système de distribution d'un gaz respiratoire froid (100) selon la revendication 1, le dispositif d'injection comprenant un appareil à raser la glace.

11. Système de distribution d'un gaz respiratoire froid (100) selon la revendication 1, le dispositif d'injection comprenant une buse à jets tourbillonnants.

12. Système de distribution d'un gaz respiratoire froid (100) selon la revendication 1, le dispositif d'injection comprenant une buse à aiguille d'impact.

13. Système de distribution d'un gaz respiratoire froid (100) selon la revendication 1, le dispositif d'injection comprenant une buse à jets en collision.

14. Système de distribution d'un gaz respiratoire froid (100) selon la revendication 1, le dispositif d'injection comprenant un générateur à brouillard de groupe de buses MEMS.
